# EUROPÄISCHE PATENTSCHRIFT

(11) **EP 1 373 543 B1**
(45) Veröffentlichungstag und Bekanntmachung des Hinweises auf die Patenterteilung: **03.02.2010**
(21) Anmeldenummer: 01984746.6
(22) Anmeldetag: 21.11.2001
(51) Int. Cl.: C12P 19/04, C08B 37/06, A23L 1/0524, A61K 31/715

(54) **VERFAHREN ZUR HERSTELLUNG VON PEKTINHYDROLYSEPRODUKTEN**
METHOD FOR PRODUCING PECTIN HYDROLYSIS PRODUCTS
PROCEDE DE FABRICATION DE PRODUITS D'HYDROLYSE DE LA PECTINE

(30) Priorität: 22.11.2000 DE 10057976
(43) Veröffentlichungstag der Anmeldung: 02.01.2004
(60) Teilanmeldung: 07022824.2
(73) Patentinhaber: N.V. Nutricia, 2712 HM Zoetermeer (NL)
(72) Erfinder: KUNZ, Markwart, 67550 Worms (DE); MUNIR, Mohammad, 67271 Kindenheim (DE); VOGEL, Manfred, 67271 Neuleiningen (DE)
(74) Vertreter: Schrell, Andreas
(86) Internationale Anmeldenummer: PCT/EP2001/013508
(87) Internationale Veröffentlichungsnummer: WO 2002/042484

(56) Entgegenhaltungen:
- EP-B- 0 716 605
- DE-A- 4 223 613
- US-A- 5 834 442
- ENDRESS H-U ET AL: "MONITORING THE COURSE OF ENZYMIC DEGRADATION OF PECTIC SUBSTANCES BY AUTOMATED FAST ION CHROMATOGRAPHY FIC" LEBENSMITTEL-WISSENSCHAFT & TECHNOLOGIE, Bd. 24, Nr. 1, 1991, Seiten 80-85, XP009003140 ISSN: 0023-6438
- KESTER H C ET AL: "Performance of selected microbial pectinases on synthetic monomethyl-esterified di- and trigalacturonates." THE JOURNAL OF BIOLOGICAL CHEMISTRY. UNITED STATES 24 DEC 1999, Bd. 274, Nr. 52, 24. Dezember 1999 (1999-12-24), Seiten 37053-37059, XP002225691 ISSN: 0021-9258

## Beschreibung

Die Erfindung betrifft ein Verfahren zur Herstellung von Pektinhydrolysaten, insbesondere eines pharmazeutischen oder diätetischen Präparats zur Verminderung und/oder Verhinderung der Adhäsion pathogener Substanzen und Organismen an eukaryontische Zellen, insbesondere Säugerzellen, oder zur Hemmung von Galectin 3-vermittelten Zell-Zell-und/oder Zell-Matrix-Wechselwirkungen, die zur Entwicklung von Tumorerkrankungen führen, Verfahren zur Blockierung der Anlagerung pathogener Substanzen oder Organismen an eukaryontische Zellen, Verfahren zur Hemmung Galectin-3 vermittelter Zell-Zell- und/oder Zell-Matrix-Wechselwirkungen, sowie die mittels dieser Verfahren hergestellten Pektinhydrolysate und Präparate.

Pathogene Organismen aber auch zellschädigende Substanzen müssen sich erst an der Oberfläche der Zielzelle anheften, um eine Infektion beziehungsweise Schädigung der angegriffenen Zelle hervorrufen zu können. Diese Anheftung oder Adhäsion wird zum Beispiel durch eine Ligand-Rezeptor-Beziehung, wobei Glykostrukturen eine wichtige Rolle spielen, vermittelt. Werden diese Glykostrukturen auf der Zielzellen-Oberfläche oder am Liganden blockiert, kann eine Infektion verhindert werden.

Glykostrukturen spielen auch eine wichtige Rolle bei der Tumorbildung und Metastasenbildung (Liotta et al., Annu. Rev. Cell Biol., 55 (1986), 1037-1057). Die Bildung von Tumoren umfasst zelluläre Interaktionen, die durch Zelloberflächenbestandteile, insbesondere Kohlenhydrat-bindende Proteine vermittelt werden. So wird die Adhäsion von Tumorzellen durch zelluläre Adhäsionsmoleküle vermittelt. Auch viele Stufen der Metastasenbildung umfassen Zell-Zell-Wechselwirkungen beziehungsweise Wechselwirkungen zwischen Zellen und der extrazellulären Matrix (ECM), die durch Zelloberflächenbestandteile vermittelt werden. Die extrazelluläre Matrix (ECM) besteht hauptsächlich aus Laminin, Fibronectin und Proteoglykanen, von denen sehr viele glykosyliert sind und deren Oligosaccharid-Seitenketten Erkennungs-Determinanten für zelluläre Adhäsionsmoleküle bieten. Laminin ist ein N-verbundenes Glycoprotein, das Poly-N-Acetyllactosamin-Sequenzen aufweist. Die Absiedelung von Metastasen erfolgt, wenn zirkulierende Agglomerate von Tumorzellen, Thrombozyten und Lymphozyten in Kapillaren über Adhäsionsmoleküle Kontakt zum Endothel aufnehmen. Diese Kontaktaufnahme liefert das Signal zur Öffnung der endothelialen Zell-Funktionen. Dadurch können die Tumorzellen über weitere Adhäsionsmoleküle an Rezeptoren auf der Basalmembran binden. Nach Zerstörung der Basalmembran erhalten die Tumorzellen direkten Zugang zum Stoma, wobei wiederum wie bei der primären Tumorinvasion Interaktionen zwischen Laminin und Fibronektin und den entsprechenden Rezeptoren erfolgen.

Wichtige Vertreter der Kohlenhydrat-bindenden Proteine sind die Galactosid-bindenden Lektine Galectin-1 und Galectin-3 (Raz und Lotan, Cancer Metastasis Rev. 6 (1987), 433; Gabius, Biochim. Biophys. Acta, 1071 (1991), 1). Von Galectin 3 ist bekannt, dass es die embolische Tumorversprengung im Blutkreislauf fördert und die Metastasenbildung erhöht. Galectin-3 wird auf der Zelloberfläche vieler Tumorzellen exprimiert, wobei sich die Galectin-3-Expression mit fortschreitender Tumorentwicklung erhöht. Galectin-3 wird ebenfalls von aktivierten Makrophagen und onkogen transformierten Zellen beziehungsweise Metastasenzellen exprimiert. Galectin-3 besitzt hohe Affinität zu Oligosacchariden, die Polylactosamine umfassen, wobei Galectin-3 insbesondere an zwei Glycoproteine bindet, die in mehreren Zelltypen, beispielsweise menschlichen Dickdarmkarzinom-Zellen und menschlichen Mammakarzinom-Zellen vorkommen. Ein anderer Ligand für Galectin 3 ist beispielsweise Laminin. Galectin 3, das auch auf der Oberfläche von Endothelzellen exprimiert wird, ist ebenfalls an der Adhäsion von Tumorzellen an Endothelzellen beteiligt.

Die US 5,834,442 beschreibt Verfahren zur Behandlung von Krebserkrankungen in Säugetieren, insbesondere zur Behandlung von Prostatakarzinomen, wobei die Behandlung von Krebserkrankungen einschließlich der Hemmung von Metastasenbildung durch orale Verabreichung von modifiziertem Pektin, vorzugsweise wasserlöslichem pH-modifiziertem Citruspektin erfolgt. Zur Herstellung von pH-modifiziertem Pektin wird eine Pektinlösung durch Erhöhung des pH-Wertes auf 10,0 und danach Absenken des pH-Wertes auf 3,0 depolymerisiert. Das modifizierte Pektin besitzt ein Molekulargewicht von etwa 1 bis 15 kd. Ratten, denen modifiziertes Citruspektin im Trinkwasser verabreicht wurden, zeigten im Vergleich zu unbehandelten Kontrollgruppen eine signifikant verringerte Bildung von Lungenmetastasen. In vitro-Experimenten zeigten, dass die Adhäsion von Galectin-3 exprimierenden MLL-Endothelzellen an Aorta-Endothelzellen der Ratte (RAEC) in Gegenwart von modifiziertem Citruspektin fast vollständig inhibiert wurde. In weiteren Experimenten wurde die Wirkung von pH-modifiziertem Citruspektin auf die Koloniebildung von MLL-Endothelzellen untersucht. Die Fähigkeit von Zellen zum Wachstum in halbfestem Medium (Anchorage-Unabhängigkeit) kann als Kriterium für die Zelltransformation und das Invasionspotential von Zellen verwendet werden, da das Zell-Wachstum in einem halbfesten Medium die Migration der Zellen und die Bildung von Kolonien erfordert. Dabei stellte sich heraus, dass modifiziertes Citruspektin sowohl die Anzahl der gebildeten MLL-Kolonien als auch deren Größe signifikant reduzieren konnte. Modifiziertes Citruspektin scheint dabei eher eine cytostatische Wirkung auszuüben als eine cytotoxische Wirkung. Auch die Wirkung von modifiziertem Citruspektin auf Zell-Zell-Wechselwirkungen und Zell-Matrix-Wechselwirkungen, die auf Kohlenhydrat-vermittelten Mechanismen, insbesondere Galectin-3-vermittelten Interaktionen, beruhen, wurde untersucht. Dabei zeigt es sich, dass modifiziertes Citruspektin im Gegensatz zu nicht-modifiziertem Citruspektin die Adhäsion von B16-F1-Melanomzellen an Laminin inhibierte. Von Laminin ist bekannt, dass es als Ligand für lösliches Galektin-3 dient.

Aus der EP 0 716 605 B1 ist bekannt, dass durch eine besonders hergestellte Karottensuppe, Blasentee, Kokosmilch etc., die Adhärenz pathogener Keime, wie etwa E. coli, an Zellen, insbesondere an Epithelzellen des Gastrointestinal- und Urogenitaltrakts wesentlich (das heißt bis zu 90 %) reduziert werden kann. Nach dieser Druckschrift ist diese Wirkung auf die in den Pflanzenprodukten vorhandenen Pektine zurückzuführen, bei denen es sich im Wesentlichen um Ketten von 1,4-α-glycosidisch verbundenen Galakturoniden handelt, deren Säuregruppen zu 20 bis 80 % mit Methanol verestert sind, und die neben Galakturonsäure gegebenenfalls noch andere Zuckerbausteine, zum Beispiel Glucose, Galactose, Xylose und Arabinose enthalten können. Aus der Druckschrift kann man weiter entnehmen, dass Monogalakturonsäure keine Blockierung der Adhäsion zeigt, während mit Digalakturonid und Trigalakturonid eine Blockierung zu 91,7 % beziehungsweise 84,6 % festzustellen ist. In dieser Druckschrift wird eindeutig festgestellt, dass die monomere Galakturonsäure keine Blockierung der Adhäsion aufweist und die erwünschte Blockierungswirkung mit zunehmendem Molekulargewicht der Galakturonide abnimmt. Daraus ergibt sich, dass der Polymerisationsgrad der erwünschten Galakturonide bei DP 2 beziehungsweise 3 liegt. Außerdem wird gefordert, dass der Veresterungsgrad <2% beträgt. Die nach der dort beschriebenen Methode hergestellten Pektinhydrolyseprodukte enthalten jedoch nur sehr niedrige Anteile an den als wirksam bezeichneten Di- und Trigalakturoniden (ca. 12% bezogen auf den Rohstoff). Diese Herstellmethode verschwendet Ressourcen und führt zu Umweltproblemen, da die in hohen Anteilen anfallenden nicht brauchbaren Nebenprodukte entsorgt werden müssen.

Weitere Galacturonide beschreiben Kester et al. (J. Biol. Chem., 274 (1999), 37053 - 37059) und Endreß et al. (Lebensm.-Wiss. u. -Technol., 24 (1991), 80 - 85). Eine biologische Wirkung dieser Galacturonide ist nicht beschrieben.

Das der vorliegenden Erfindung zugrunde liegende technische Problem besteht darin, weitere Verfahren und Mittel zur Bekämpfung von Infektionen und zur Verminderung und/oder Verhinderung der Adhäsion von schädlichen, insbesondere pathogenen, Substanzen und Organismen an eukaryontische Zellen, insbesondere Säugerzellen, sowie zur Blockierung von Wechselwirkungen zwischen Säugerzellen, insbesondere Tumorzellen, die durch auf der Zelloberfläche befindliche Kohlenhydrat-bindende Galectin 3-Moleküle vermittelt werden und für die Entwicklung von insbesondere Tumorerkrankungen verantwortlich sind, und zur Behandlung von Tumorerkrankungen, insbesondere zur Verhinderung von Metastasenbildungen, bei Säugern bereitzustellen.

Dieses technische Problem wird durch die erfindungsgemäß vorgesehenen Verfahren zur Herstellung von Pektinhydrolyseprodukten gelöst, die zur Herstellung von Oligogalakturoniden mit einem möglichst geringen Anteil an Monomeren, einem hohen Anteil von Molekülen mit jeweils zumindest einer Doppelbindung sowie mit einem Veresterungsgrad von ≥20% führen und die mit einer wesentlich höheren Ausbeute als im Stand der Technik beschrieben durchgeführt werden können.

Das Problem wird insbesondere dadurch gelöst, dass man eine wässrige Lösung oder Suspension eines Pektins oder pektinhaltigen, insbesondere pflanzlichen, Materials, vorzugsweise eines Pektins mit einem hohen Veresterungsgrad, in einem ersten Verfahrensschritt mit einem ersten pektinhydrolysierenden Enzym A und dann in einem zweiten Verfahrensschritt mit einem zweiten pektinhydrolysierenden Enzym B behandelt. Man erhält ein vorstehend definiertes Pektinhydrolyseprodukt, welches hervorragende Eigenschaften als Mittel zur Verminderung oder Verhinderung der Adhäsion für die Lebens- und/oder Vermehrungsfunktionen von Zellen schädlicher, zum Beispiel pathogener, allergener, infektiöser oder toxisches, Substanzen oder Organismen, zum Beispiel Mikroorganismen wie Hefen, Pilze, Keime, Bakterien, Viren, Sporen, Viroide, Prionen aufweist.

Bei dem verwendeten Enzym A kann es sich zum Beispiel um eine Pektinlyase (EC 4.2.2.10) oder eine Endopolygalakturonase (EC 3.2.1.15), bevorzugt jedoch um eine Pektinlyase handeln. Bei dem verwendeten Enzym B kann es sich um eine Endopolygalakturonase oder eine Pektinlyase, bevorzugt jedoch um eine Endopolygalakturonase handeln.

Erfindungsgemäß wurde überraschenderweise festgestellt, dass Galakturonide, die Doppelbindungen im Molekül tragen bei der Blockierung der Adhäsion von zum Beispiel pathogenen Keimen und zellschädigenden Substanzen an Epithelzellen des Gastrointestinal-und Urogenitaltraktes besonders wirksam sind. Außerdem ist für eine besonders effiziente Verhinderung und/oder Verminderung, zum Beispiel Blockierung, ein höherer Veresterungsgrad, insbesondere ≥20, vorzugsweise ≥30, ≥40, ≥50, besonders bevorzugt ≥60, ≥65, ≥70 oder ≥71% erforderlich. Die im Stand der Technik beschriebene Arbeitsweise führt jedoch nur zu Galakturoniden, die keine Doppelbindungen aufweisen und praktisch vollständig entestert sind.

Unter dem Begriff Veresterungsgrad wird im Zusammenhang mit der vorliegenden Erfindung der Anteil der grundsätzlich für eine Veresterung zur Verfügung stehenden Säuregruppen eines Galakturonids verstanden, der mit einem Alkohol, insbesondere Methanol verestert ist.

Im Zusammenhang mit der vorliegenden Erfindung werden unter ungesättigten Galakturonsäuremoleküle insbesondere 4,5-ungesättigte Galakturonsäuremoleküle verstanden.

In einer Ausführungsform der Erfindung ist es vorgesehen, nach der Behandlung mit dem Enzym B eine Behandlung mit einem weiteren, dritten Enzym C anzuschließen. Bei dem hierbei verwendeten Enzym C kann es sich um eine Pektinesterase (EC 3.1.1.11) handeln. Damit kann der Veresterungsgrad der Produkte besonders gezielt eingestellt werden.

In einer weiteren Ausführungsform der Erfindung ist es vorgesehen, dass nach erfolgter erfindungsgemäßer enzymatischer Behandlung die verbliebenen ungelösten Bestandteile aus der Lösung durch Zentrifugation und/oder Ultrafiltration abgetrennt werden.

In einer weiteren Ausführungsform ist es vorgesehen, dass die nach erfolgter erfindungsgemäßer Enzymbehandlung und Klärung durch Zentrifugation beziehungsweise Ultrafiltration erhaltene Lösung durch eine der an sich bekannten Methoden in trockene Form, zum Beispiel in gemahlene, körnige, granulierte oder Pulverform überführt wird.

Die nach der erfindungsgemäßen enzymatischen Behandlung erhaltene Lösung oder das daraus gewonnene trockene Produkt zeigen sehr gute Wirkung im Hinblick auf die Blockierung der Adhäsion von zum Beispiel pathogenen Keimen und zellschädigenden Substanzen an zum Beispiel Epithelzellen des Gastrointestinal- und Urogenitaltraktes bei Mensch und Tier. Sie können deshalb zum Beispiel in Tierfutter zum Beispiel zur Verhinderung von Durchfallerkrankungen bei der Ferkelaufzucht eingesetzt werden.

Die erfindungsgemäß eingesetzte Lösung oder Suspension des Pektins beziehungsweise des pektinhaltigen, vorzugsweise pflanzlichen, Materials weist einen pH-Wert in einem Bereich von 3,5 bis 5,5 oder/und in einer weiteren bevorzugten Ausführungsform eine Konzentration des Pektins von 3 % bis 25 % auf.

Die Behandlungen mit den Enzymen A, B und gegebenenfalls C finden bei einem pH-Wert von 3,5 bis 5,5 über eine Dauer von 2 Stunden bis 24 Stunden bei einer Temperatur von 25°C bis 60°C und einer Konzentration des Enzyms A, B gegebenenfalls C von 10 bis 30 ml/kg Pektin statt.

In einer weiteren bevorzugten Ausführungsform ist vorgesehen, dass der Anteil der Galakturonide am Pektinhydrolysat (Gew.-% in Bezug auf Trockensubstanz) mindestens 60, ≥70, ≥75, ≥80 oder besonders bevorzugt mindestens 85 Gew.-% beträgt.

In einer weiteren bevorzugten Ausführungsform ist vorgesehen, dass in dem Pektinhydrolysat der Anteil von Kohlenhydraten mit einem DP-1(Monomere) an den Gesamtkohlenhydraten des Pektinhydrolysats <25, <20, <10, <8, <5, besonders bevorzugt <4 Gew.-% (bezogen auf Trockensubstanz) ist.

In einer weiteren bevorzugten Ausführungsform ist vorgesehen, dass der Anteil der Kohlenhydrate, insbesondere Galakturonide mit einem Polymerisationsgrad DP >10 bezogen auf den Gesamtkohlenhydratgehalt des Pektinhydrolysats weniger als 10, <8, besonders bevorzugt <5 Gew.-% (bezogen auf Trockensubstanz) beträgt.

In einer weiteren bevorzugten Ausführungsform ist vorgesehen, dass der Anteil der ungesättigten Galakturonide am Gesamtgehalt der Galakturonide im Pektinhydrolysat mindestens 10, vorzugsweise >15, >20, >25, >30, insbesondere 36,5 Gew.-% bis 46 Gew.-% (bezogen auf Trockensubstanz) beträgt.

Die erfindungsgemäß hergestellten Pektinhydrolysate weisen in bevorzugter Ausführung einen Anteil von mindestens 60, ≥70, ≥75, ≥80 oder besonders bevorzugt mindestens 85 % (auf Trockensubstanz) an Kohlenhydraten, insbesondere Galakturoniden mit einen Polymerisationsgrad von 2 bis 10, bevorzugt von 3 bis 8, besonders bevorzugt 4,5 auf (Gew.-% Trockensubstanz, bezogen auf Gesamtkohlenhydratgehalt des Pektinhydrolysats).

Das eingesetzte Pektin ist in besonders bevorzugter Ausführungsform Citruspektin, Apfelpektin oder Zuckerrübenpektin.

Das eingesetzte pektinhaltige Material, insbesondere pflanzliche pektinhaltige Material, ist in bevorzugter Ausführungsform Apfeltrester, Zuckerrübenschnitzel oder Citrus-Pellets, also getrocknete Rückstände, zum Beispiel aus der Orangensaft-, Zitronensaft- und/oder Limonensaft-Herstellung.

Die erfindungsgemäß hergestellten Pektinhydrolysate, also Pektinhydrolyseprodukte, eignen sich in hervorragender Weise als pharmazeutisches beziehungsweise diätetisches Präparat zur Bekämpfung von Infektionskrankheiten oder/und zur Bekämpfung der Anlagerung von schädlichen Substanzen und/oder Organismen an Säugerzellen, insbesondere menschliche Zellen.

Die erfindungsgemäß hergestellten Pektinhydrolysate eignen sich ebenfalls hervorragend als pharmazeutisches Präparat zur Hemmung von Zell-Zell-Wechselwirkungen und/oder Wechselwirkungen zwischen Zellen und der extrazellulären Matrix bei einem Menschen oder einem Säugetier, insbesondere solchen Wechselwirkungen, die durch auf der Zelloberfläche befindliche Galectin 3-Moleküle vermittelt werden. Die erfindungsgemäßen Pektinhydrolyseprodukte sind daher insbesondere zur Hemmung von Zell-Zell-und/oder Zell-Matrix-Wechselwirkungen geeignet, an denen Tumorzellen beteiligt sind und die daher für die Entwicklung von Erkrankungen, insbesondere Tumorerkrankungen bei einem Menschen oder einem Säugetier verantwortlich sind. Die erfindungsgemäßen Pektinhydrolysate sind daher auch als pharmazeutisches Präparat zur Behandlung von Tumorerkrankungen, insbesondere zur Reduzierung des Tumorwachstums und/oder zur Reduzierung der Metastasenbildung bei Krebserkrankungen, geeignet, da sie die Galectin-3-vermittelte Tumorzelladhäsion und/oder das Invasionspotential der Tumorzellen verhindern.

Die Erfindung betrifft daher auch die erfindungsgemäß erhaltenen und einen Anteil von Kohlenhydraten mit einem DP-1 an den Gesamtkohlenhydraten des Pektinhydrolysats von weniger als 25 Gew.-% (bezogen auf die Trockensubstanz) aufweisenden Pektinhydrolysate, also Pektinhydrolyseprodukte, enthaltende pharmazeutische Präparate und diätetische Präparate, die beispielsweise als Lebensmittel oder Genussmittel ausgeführt sein können, zum Beispiel Milchprodukte, Joghurt, Cerealien, Backwaren etc..

Die Erfindung betrifft auch die Verwendung der vorgenannten Pektinhydrolyseprodukte zur Herstellung von Arzneimitteln zur Verhinderung der Anlagerung oder Adhäsion von schädlichen Substanzen und/oder Organismen an Säugerzellen, insbesondere menschliche Zellen, insbesondere zur Bekämpfung, das heißt Prophylaxe und Therapie, von Infektionskrankheiten, Vergiftungen, Allergien etc.

Die Erfindung betrifft auch die Verwendung der vorgenannten Pektinhydrolyseprodukte zur Verhinderung der Anlagerung oder Adhäsion von schädlichen Substanzen und/oder Organismen an Säugerzellen, insbesondere menschlichen Zellen, insbesondere zur Bekämpfung von Infektionskrankheiten, Vergiftungen, Allergien etc..

Die erfindungsgemäß bekämpften Infektionen können insbesondere Infektionen des Blutsystems, der Atemwege, des Urogenitaltraktes, des Nasen-Rachenraumes oder des Gastrointestinaltraktes sein.

Ein weiteres Einsatzgebiet ist in der Humanernährung, wo sie zum Beispiel bei der Verhinderung von Durchfallerkrankungen bei Säuglingen aber auch Erwachsenen helfen.

Die Erfindung betrifft auch die Verwendung der vorgenannten Pektinhydrolyseprodukte zur Hemmung von Zell-Zell-Wechselwirkungen und/oder Wechselwirkungen zwischen Zellen und der extrazellulären Matrix, insbesondere solcher Wechselwirkungen, die durch auf der Zell-Oberfläche befindliche Kohlenhydrat-bindende Galectin 3-Moleküle vermittelt werden und die für die Entwicklung humaner oder Säugetier-Erkrankungen, insbesondere Tumorerkrankungen, verantwortlich sind. Bei diesen Erkrankungen handelt es sich insbesondere um Prostata-Karzinome, Nieren-Karzinome, Kaposi-Sarkom, Verlaufsformen der chronischen Leukämie, Mammakarzinome, Mamma-Adenokarzinome, Sarkome, Ovarialkarzinome, Rektumkarzinome, Rachenkarzinome, Melanome, Dünndarm-Tumore, Dickdarm-Karzinome, Blasentumore, Mastozytome, Lungenkarzinome, Bronchialkarzinome, Rachen-Plattenepithelkarzinome, Gastrointestinalkarzinome und Magenkarzinome. Die erfindungsgemäßen Pektinhydrolyseprodukte können insbesondere zur Reduzierung des Invasionspotentials metastasierender Tumorzellen und/oder zur Hemmung der Adhäsion von Tumorzellen verwendet werden. Vorzugsweise werden die erfindungsgemäß hergestellten Pektinhydrolyseprodukte oral verabreicht.

Eine weitere bevorzugte Ausführungsform der Erfindung betrifft die Verwendung der erfindungsgemäßen Pektinhydrolyseprodukte zur Behandlung von Tumorerkrankungen eines Menschen oder eines Säugetiers. Die erfindungsgemäßen Pektinhydrolysate lassen sich vorzugsweise zur Behandlung solcher Tumore einsetzen, die auf Zell-Zell-Wechselwirkungen und/oder Wechselwirkungen zwischen Zellen und der extrazellulären Matrix beruhen, insbesondere solchen Wechselwirkungen, die durch auf der Zelloberfläche befindliche Kohlenhydrat-bindende Galectin-3-Moleküle vermittelt werden. Unter Verwendung der erfindungsgemäßen Pektinhydrolysate können daher vorzugsweise Prostata-Karzinome, Nieren-Karzinome, Kaposi-Sarkom, Verlaufsformen der chronischen Leukämie, Mammakarzinome, Mamma-Adenokarzinome, Sarkome, Ovarialkarzinome, Rektumkarzinome, Rachenkarzinome, Melanome, Dünndarm-Tumore, Dickdarm-Karzinome, Blasentumore, Mastozytome, Lungenkarzinome, Bronchialkarzinome, Rachen-Plattenepithelkarzinome, Gastrointestinalkarzinome und Magenkarzinome behandelt werden. Die Verwendung der erfindungsgemäßen Pektinhydrolysate zur Tumorbehandlung zielt insbesondere auf die Hemmung der Adhäsion von Tumorzellen und/oder die Verminderung des Invasionspotentials metastasierender Tumorzellen ab.

Die Erfindung betrifft ebenfalls die Verwendung der vorgenannten Pektinhydrolyseprodukte zur Herstellung eines pharmazeutischen Präparates zur Hemmung von Zell-Zell-Wechselwirkungen und/oder Wechselwirkungen zwischen Zellen und der extrazellulären Matrix, insbesondere solcher Wechselwirkungen, die durch auf der Zell-Oberfläche befindliche Kohlenhydrat-bindende Galectin 3-Moleküle vermittelt werden und die für die Entwicklung humaner oder Säugetier-Erkrankungen, insbesondere der vorstehend beschriebenen Tumorerkrankungen, verantwortlich sind. Das pharmazeutische Präparat zur Hemmung von Zell-Zell-Wechselwirkungen und/oder Wechselwirkungen zwischen Zellen und der extrazellulären Matrix wird vorzugsweise oral verabreicht.

Eine weitere bevorzugte Ausführungsform der Erfindung betrifft daher auch die Verwendung der erfindungsgemäßen Pektinhydrolyseprodukte zur Herstellung eines pharmazeutischen Präparates, das zur Behandlung der vorstehend beschriebenen Tumorerkrankungen verwendet werden kann, also zur Behandlung von Tumoren, die auf Zell-Zell-Wechselwirkungen und/oder Wechselwirkungen zwischen Zellen und der extrazellulären Matrix beruhen, insbesondere solcher Wechselwirkungen, die durch auf der Zell-Oberfläche befindliche Kohlenhydrat-bindende Galectin 3-Moleküle vermittelt werden. Erfindungsgemäß ist vorgesehen, dass das pharmazeutische Präparat zur Reduzierung des Tumorwachstums und/oder zur Reduzierung der Metastasenbildung eingesetzt werden kann, wobei das erfindungsgemäße pharmazeutische Präparat insbesondere die Adhäsion von Tumorzellen verhindert und/oder das Invasionspotential von Tumorzellen reduziert. Vorzugsweise wird das erfindungsgemäße pharmazeutische Präparat oral verabreicht.

Die vorliegende Erfindung betrifft ebenfalls ein Verfahren zur Blockierung der Anlagerung von schädlichen, insbesondere pathogenen Substanzen oder Organismen an Zellen eines menschlichen oder Säugetierkörpers, umfassend die Verabreichung der erfindungsgemäß hergestellten Pektinhydrolyseprodukte an einen Menschen oder ein Säugetier in einer Menge, die ausreicht, die Anlagerung der schädlichen Substanzen oder Organismen an Säugerzellen zu blockieren und die Entstehung einer Infektion zu verhindern. Vorzugsweise werden die erfindungsgemäß hergestellten Pektinhydrolyseprodukte oral verabreicht.

Die Erfindung betrifft ebenfalls ein Verfahren zur Hemmung von Zell-Zell-Wechselwirkungen und/oder Wechselwirkungen zwischen Zellen und der extrazellulären Matrix, die durch auf der Zelloberfläche befindliche Kohlenhydrat-bindende Galectin-3-Moleküle vermittelt werden und für die Entwicklung humaner oder Säugetier-Erkrankungen, insbesondere die vorstehend genannten Tumorerkrankungen verantwortlich sind, umfassend die Verabreichung der erfindungsgemäß hergestellten Pektinhydrolyseprodukte an einen Menschen oder ein Säugetier mit einer Tumorerkrankung in einer Menge, die ausreicht, Galectin-3-vermittelte Zell-Zell- und/oder Zell-Matrix-Wechselwirkungen zu reduzieren und/oder zu hemmen. Vorzugsweise werden die erfindungsgemäß hergestellten Pektinhydrolyseprodukte oral verabreicht.

Die vorliegende Erfindung betrifft auch pharmazeutische Präparate, die die erfindungsgemäßen Pektinhydrolyseprodukte in pharmazeutisch oder therapeutisch wirksamen Mengen enthalten. Im Zusammenhang mit der vorliegenden Erfindung wird unter einem "pharmazeutischen Präparat" ein zu diagnostischen, therapeutischen und/oder prophylaktischen Zwecken verwendetes Gemisch verstanden, das die erfindungsgemäßen Pektinhydrolyseprodukte als Wirkstoffe in einer bei einem Patienten oder einem Säugetier gut applizierbaren Form enthält. Das pharmazeutische Präparat kann sowohl ein festes als auch ein flüssiges Gemisch sein. "In pharmazeutisch oder therapeutisch wirksamen Mengen" bedeutet, dass die in dem pharmazeutischen Präparat enthaltenen Wirkstoffe in einer Dosis enthalten sind, die ausreicht, den Ausbruch einer Erkrankung, beispielsweise einer Infektionskrankheit oder einer Tumorerkrankung zu verhindern, den Zustand einer derartigen Erkrankung zu heilen, die Progression einer solchen Erkrankung zu stoppen und/oder die Symptome einer solchen Erkrankung zu lindern. Die erfindungsgemäßen pharmazeutischen Präparate weisen zusätzlich zu den erfindungsgemäßen Pektinhydrolyseprodukten in bevorzugter Ausführungsform pharmazeutische verträgliche Träger sowie gegebenenfalls Verdünnungsmittel, Trennmittel, Gleitmittel, Hilfsstoffe, Füllstoffe, Süßstoffe, Aromastoffe, Farbstoffe, Geschmacksstoffe oder andere pharmazeutisch wirksame Substanzen auf.

In den erfindungsgemäß vorgesehenen diätetischen Präparaten werden die Pektinhydrolyseprodukte ebenfalls in einer pharmazeutisch wirksamen Menge eingesetzt.

Weitere bevorzugte Ausführungsformen sind in den Unteransprüchen aufgeführt.

Die Erfindung wird mit Hilfe der folgenden Beispiele näher erläutert.

### Beispiel 1:

Zu 1 1 einer Citruspektinlösung (30 g hochverestertes Pektin in 1 l Wasser) wurde 0,3 ml einer Pektinlyase (zum Beispiel Rohapect PTE von Röhm) zugegeben und die Lösung unter Rühren bei pH 5,0 und 45°C für 2 Stunden inkubiert. Dann wurden 0,75 ml einer Endopolygalakturonase (zum Beispiel Pectinase PL von Amano) hinzugefügt und bei unveränderten Reaktionsbedingungen für weitere 3 Stunden inkubiert. Dann wurden die Enzyme durch Erhitzung auf 95°C inaktiviert. Der unlösliche Rückstand wurde durch Zentrifugation entfernt, die klare Lösung zur Trockne eingedampft und der erhaltene Feststoff gewogen. Die Auswaage betrug 25,8 g (entsprechend einer Ausbeute von 75,6 % bezogen auf den eingesetzten Rohstoff).

Das erhaltene Produkt wurde nach allgemein bekannten Analysemethoden untersucht und dafür folgende Zusammensetzung festgestellt:

| | | | | | |
|---|---|---|---|---|---|
| Kohlenhydrate | | | DP 1 | | 3,6 % |
| | Galakturonide | | | | 83,9 % |
| | davon ungesättigte | | | 46,0 % | |
| | (angenommener mittl. DP= 4,5) | | | | |
| | | DP 2 - 10 | | 80,4 % | |
| | | DP > 10 | | 16,0 % | |
| Veresterungsgrad | | | | 72,0 % | |
| Salzgehalt | | | | | 3,0 % |
| Rohprotein | | | | | 1,7 % |
| Wassergehalt | | | | | 4,6 % |

### Beispiel 2:

Zu 1 l einer Citruspektinlösung (30 g hochverestertes Pektin in 1 l Wasser) wurde 0,3 ml einer Pektinlyase (zum Beispiel Rohapect PTE von Röhm) zugegeben und die Lösung unter Rühren bei pH 5,0 und 45°C für 2 Stunden inkubiert. Dann wurden 0,75 ml einer Endopolygalakturonase (zum Beispiel Pectinase PL von Amano) hinzugefügt und bei unveränderten Reaktionsbedingungen für weitere 3 Stunden inkubiert. Dann wurden die Enzyme durch Erhitzung auf 95°C inaktiviert.

Der unlösliche Rückstand wurde durch Zentrifugation entfernt und die klare Lösung wurde ultrafiltriert (cut-off 10.000). Das Permeat wurde getrocknet und ergab 22,8 g Feststoff (Ausbeute 66,8 % bezogen auf den eingesetzten Rohstoff).

| | | | | | |
|---|---|---|---|---|---|
| Kohlenhydrate | | | DP 1 | | 3,0 % |
| | Galakturonide | | | | 84,1 % |
| | davon ungesättigte | | | 36,5 % | |
| | (angenommener mittl. DP = 4,5) | | | | |
| | | DP 2 - 10 | | 93,0 % | |
| | | DP > 10 | | 4,0 % | |
| Veresterungsgrad | | | | 72,0 % | |
| Salzgehalt | | | | | 6,7 % |
| Rohprotein | | | | | 1,3 % |
| Wassergehalt | | | | | 4,4 % |

### Beispiel 3

Zu 1 l einer Citruspektinlösung (30 g hochverestertes Pektin in 1 l Wasser) wurde 0,3 ml einer Pektinlyase (zum Beispiel Rohapect PTE von Röhm) zugegeben und die Lösung unter Rühren bei pH 5,0 und 45°C für 2 Stunden inkubiert. Dann wurden 0,75 ml einer Endopolygalakturonase (zum Beispiel Pectinase PL von Amano) hinzugefügt und bei unveränderten Reaktionsbedingungen für weitere 3 Stunden inkubiert. Danach wurden 0,5 ml einer Pektinesterase (zum Beispiel Rheozyme von Novo Nordisk) hinzugefügt und für weitere 45 Minuten inkubiert. Dann wurden die Enzyme durch Erhitzung auf 95°C inaktiviert. Der unlösliche Rückstand wurde durch Zentrifugation entfernt, die klare Lösung zur Trockne eingedampft.

Das erhaltene Produkt wurde nach allgemein bekannten Analysemethoden untersucht. Es wurde abweichend vom Beispiel 1 ein Veresterungsgrad von 35 % festgestellt.

### Beispiel 4:

Getrocknete Orangenschalen oder Citrus-Pellets wurden auf eine Partikelgröße von ca. 1-5 mm zerkleinert und davon 100 g in 400 ml Wasser eingerührt und aufquellen gelassen. Dann wurde konzentrierte Salpetersäure (10 g) hinzugefügt und die Suspension auf 85°C erwärmt und bei dieser Temperatur für 1,5 Stunden gerührt. Dann wurde auf 45°C abgekühlt, der pH-Wert mit NaOH auf 4,5 angehoben und nach Zugabe von 0,3 ml einer Pektinlyase (zum Beispiel Rohapect PTE von Röhm) für 2 Stunden inkubiert. Dann wurden 0,75 ml einer Endopolygalakturonase (zum Beispiel Pectinase PL von Amano) hinzugefügt und bei unveränderten Reaktionsbedingungen für weitere 3 Stunden inkubiert. Dann wurden die Enzyme durch Erhitzung auf 95°C inaktiviert, aufkonzentriert und die Suspension an einem Walzentrockner getrocknet.

### Beispiel 5:

Getrocknete Orangenschalen oder Citrus-Pellets wurden auf eine Partikelgröße von ca. 1-5 mm zerkleinert und davon 100 g in 400 ml Wasser eingerührt und aufquellen gelassen. Dann wurde konzentrierte HCl (8 g) hinzugefügt und die Suspension auf 85°C erwärmt und bei dieser Temperatur für 1,5 Stunden gerührt. Dann wurde auf 45°C abgekühlt, der pH-Wert mit NaOH auf 4,5 angehoben und nach Zugabe von 0,3 ml einer Pektinlyase (zum Beispiel Rohapect PTE von Röhm) für 2 Stunden inkubiert. Dann wurden 0,75 ml einer Endopolygalakturonase (zum Beispiel Pectinase PL von Amano) hinzugefügt und bei unveränderten Reaktionsbedingungen für weitere 3 Stunden inkubiert.

Dann wurden die Enzyme durch Erhitzung auf 95°C inaktiviert, aufkonzentriert und die Suspension an einem Walzentrockner getrocknet.

### Beispiel 6:

Verhinderung der Adhäsion von pathogenen Keimen in humanen Epithelzellen

Für diesen Test wurden menschliche Uroepithelzellen, gewonnen durch Zentrifugation aus dem Morgenharn sowie je zwei Stämme von Staphylococcus aureus und E. coli, jeweils als Suspension mit 10⁹ Keimen/mL eingesetzt.

### Testdurchführung

Epithelzellen und Keimsuspension wurden zusammen bei 37°C für 30 Minuten inkubiert. Dann wurden die Epithelzellen von den nicht adhärenten Keimen durch Membranfiltration (8µ) abgetrennt. Die Filter wurden mehrfach gewaschen, in physiologische Kochsalzlösung eingebracht und die Epithelzellen darin suspendiert. Nach Zentrifugation der Suspension in Kochsalzlösung wurde das Pellet auf Objektträger aufgetragen, und nach May-Grünwald und Giemsa gefärbt. Die Anzahl der an 50 Epithelzellen anhaftenden Keime wurde gezählt. Die Zahl ergab den Leerwert. Als Kontrolle dienten Epithelzellen ohne Zugabe einer Keimsuspension.

In dem Hauptversuch wurden Epithelzellen zunächst mit unterschiedlich konzentrierten wässrigen Lösungen aus erfindungsgemäß hergestellten (entsprechend Beispiel 1) Pektinhydrolyseprodukten für 1, 2 beziehungsweise 3 Stunden inkubiert. Dann wurden sie mit der Keimsuspension zusammengebracht und wie oben beschrieben weiterbehandelt. Die Zählung der anhaftenden Keime an 50 Epithelzellen ergab den Messwert.

### Ergebnis

Bei den als Vergleich eingesetzten "neutralen" Kohlenhydraten wie zum Beispiel Raffinose, Nystose und Isomelezitose wurde keine Verminderung der Keimanlagerung an den Epithelzellen festgestellt. Mit den erfindungsgemäßen Pektinhydrolyseprodukten wurde die Adhäsion aller geprüfter Mikroorganismen fast vollständig (Blockierung: >95%) verhindert.

### Beispiel 7:

1,5 g des in Beispiel 2 gewonnenen getrockneten Permeates wurden in 100 ml 50 mM Na-Acetat-Lösung, pH-Wert 5,0, gelöst und anschließend über eine Säule (2,6 x 30 cm), die mit dem Anionenaustauscher AG 1 X2 (Fa. BioRad) gefüllt und mit 50 mM Na-Acetat-Lösung, pH-Wert 5,0, äquilibriert worden war, gegeben. Der Vorlauf aus der Säule wurde mittels HPAEC (Highperformance anion exchange chromatography) analysiert sowie mittels 1 N HCl bei 95°C für eine Stunde hydrolysiert.

### Ergebnis:

Im Vergleich mit Raftiline (Fa. Orafti) als Standard wiesen die von der Säule eluierten Oligosaccharide einen DP-Verteilung von 2 - 12 auf.

Die Analyse der Hydrolysate mittels eines Zuckeranalysators (Fa. Biotronik) ergab an Monosacchariden vornehmlich Galactose (70 %), daneben Arabinose (23 %) sowie Spuren an Glucose und Mannose. Insgesamt wurden 8,3 % der Galacturonide-haltigen Produkte als neutralzuckerhaltige Oligosaccharide im Vorlauf erhalten.

### Beispiel 8:

### Wachstum von Colonkarzinom-Zelllinien auf extrazellulärer Matrix (ECM) in Gegenwart von Pektinhydrolysat

Die humanen Colonkarzinom-Zelllinien HT-29 beziehungsweise Caco-2 wurden in einer Zelldichte von 1 x 10⁴ Zellen/ml auf 15 mm-Petrischalen ausgesät und im Medium RPMI 1640 + 10 % fötalem Kälberserum (FCS) (HT-29) beziehungsweise MEM + 10 % FCS (Caco-2) bei 37°C unter einer 5 % CO₂ enthaltenden Atmosphäre kultiviert. Die Zellen wurden 1 bis 2 Tage bis zur Konfluenz wachsen gelassen. Anschließend wurden die Schalen einmal mit PBS gewaschen, dann mit PBS und 0,5 % Triton X-100 30 min bei Raumtemperatur auf einer Schüttelvorrichtung inkubiert und danach 3x mit PBS gewaschen. Anschließend wurden die vorstehend beschriebenen Zelllinien wiederum auf den Schalen mit den so präparierten ECM-Schichten ausgesät. Der Einfluss von Pektinhydrolysat auf das Zellwachstum wurde durch Messung der Zellzahl bestimmt. Dazu wurden die Zellen nach 48 Stunden mittels Trypsin/EDTA-Lösung in HBSS wieder abgelöst (10 min) und in PBS-Lösung gewaschen. Anschließend wurde die Anzahl lebender Zellen durch Färben mit Tryphanblau-Lösung (650 mg Tryphanblau in 400 ml 0,9 % NaCl, 1:1 (v/v)) in einer Neubauer-Zählkammer bestimmt. Zur Kontrolle wurden Experimente mit Glucose durchgeführt. Die Ergebnisse sind in Tabelle 1 gezeigt.

Aus Tabelle 1 geht hervor, dass Glucose keinen Einfluss auf das Wachstum der Zelllinien HT 29 und Caco-2 ausübte, während Pektinhydrolysat das Wachstum der Zellen in Abhängigkeit von der eingesetzten Konzentration um bis zu 75% reduzierte.

**Tabelle 1**

| | Reduktion des Zell-Wachstums | | | |
|---|---|---|---|---|
| | HT 29 | | Caco-2 | |
| Konzentration | Pektinhydrolysat | Glucose | Pektinhydrolysat | Glucose |
| 0,01 % | 30 % | 0 % | 25 % | 0 % |
| 0,1 % | 45 % | 1 % | 43 % | 0 % |
| 1,0 % | 75 % | 0 % | 70 % | 0 % |

### Beispiel 9:

### Verminderung der Invasionskapazität von Caco-2-Zellen durch Pektinhydrolysat

Die Wirkung von Pektinhydrolysat auf die Invasionskapazität von Caco-2-Zellen wurde unter Verwendung des von Erkell und Schirrmacher beschriebenen Invasionstests (Cancer Research, 48 (1988), 6933-6937) untersucht. Der Test basiert auf der Wanderung von Zellen durch die Poren eines Nucleopore Polycarbonat-Filters in ein Proteingel, das verschiedene ECM-Proteine wie beispielsweise Collagen Typ I und III, Fibronektin und Laminin enthält, auf ein Nitrocellulosefilter. Die Zellen wurden gemeinsam mit dem Pektinhydrolysat in das Testsystem gegeben und anschließend wurden die durch die Proteinschicht gewanderten Zellen in der unteren Nitrocelluloseschicht quantitativ ausgewertet. Zur Kontrolle wurde die Wirkung von Glucose auf die Invasionskapazität von Caco-2-Zellen untersucht.

Die Ergebnisse dieser Untersuchungen sind in Tabelle 2 dargestellt. Die Ergebnisse zeigen, dass das erfindungsgemäße Pektinhydrolysat die Invasionskapazität von Caco-2-Zellen in Abhängigkeit von der eingesetzten Konzentration teilweise erheblich reduzieren konnte, während Glucose lediglich in höheren Konzentrationen eine geringfügige Verminderung der Invasionskapazität von Caco-2-Zellen bewirkte.

**Tabelle 2**

| | Verminderung der Invasionskapazität von Caco-2-Zellen | |
|---|---|---|
| Konzentration | Pektinhydrolysat | Glucose |
| 0,01 % | 30 % | 0 % |
| 0,1 % | 69 % | 2 % |
| 1,0 % | 88 % | 3 % |

### Beispiel 10:

### Anti-Galectin-3-Antikörperbindung durch Pektinhydrolysat

Die Expression von Galectin-3 auf Colon-Karzinomzellen wurde unter Verwendung eines anti-Galectin-3-spezifischen monoclonalen Antikörpers (Maus-Ig) und eines entsprechenden anti-Maus-FITCgekoppelten sekundären Antikörpers mittels Immunfluoreszenz/Durchflusscytometrie-Verfahren bestimmt. Steigende Konzentrationen des Pektinhydrolysates und von Glucose als Kontrolle wurden gemeinsam mit dem primären Antikörper auf den Zielzellen inkubiert und anschließend wurde der inhibierende Einfluss der löslichen Zuckersubstanz auf die anti-Galectin-3-Bindung gemessen.

Der Einfluss von Pektinhydrolysat auf die anti-Galectin-3-Bindung ist in Tabelle 3 gezeigt. Aus Tabelle 3 geht hervor, dass Glucose die Bindungsreaktion des monoclonalen anti-Galectin-3-Antikörpers nicht oder lediglich geringfügig reduziert, während Pektinhydrolysat die Bindung des Antikörpers in Abhängigkeit von der eingesetzten Konzentration teilweise erheblich reduziert.

**Tabelle 3**

| | Reduktion der Bindung des monoclonalen anti-Galectin-3 Antikörpers in % | | | |
|---|---|---|---|---|
| | HT-29 | | Caco-2 | |
| Konzentration | Pektinhydrolysat | Glucose | Pektinhydrolysat | Glucose |
| 0,01 % | 34 | 0 | 28 | 0 |
| 0,1 % | 67 | 0 | 63 | 0 |
| 1 % | 85 | 2 | 82 | 0 |

## Patentansprüche

1. Verfahren zur Herstellung von Pektinhydrolyseprodukten, wobei ein Pektin oder pektinhaltiges pflanzliches Material in wässriger Lösung oder Suspension in einem ersten Verfahrensschritt mit einem pektinhydrolysierenden Enzym A und in einem zweiten Verfahrensschritt mit einem pektinhydrolysierenden Enzym B behandelt wird, wobei Pektinhydrolyseprodukte erhalten werden mit einem Anteil an Galakturoniden, die zumindest ein 4,5-ungesättigtes Galakturonsäuremolekül enthalten und mit Methanol zu ≥20% verestert sind.

2. Verfahren zur Herstellung von Pektinhydrolyseprodukten nach Anspruch 1, wobei die aus dem zweiten verfahrensschritt erhaltenen flüssigen Hydrolyseprodukte in einem dritten Verfahrensschritt mit einem Enzym C behandelt werden, wobei es sich bei dem Enzym C um eine Pektinesterase (EC 3.1.1.11) handelt.

3. Verfahren zur Herstellung von Pektinhydrolyseprodukten nach Anspruch 1 oder 2, wobei die aus dem zweiten oder dritten Verfahrensschritt erhaltenen flüssigen Hydrolyseprodukte durch Filtration und/oder Zentrifugation von unlöslichen Bestandteilen befreit und in trockene Form überführt werden.

4. Verfahren nach einem der Ansprüche 1 bis 3, **dadurch gekennzeichnet, dass** es sich bei dem eingesetzten Pektin um Citruspektin, Apfelpektin oder Zuckerrübenpektin handelt.

5. Verfahren nach einem der Ansprüche 1 bis 4, **dadurch gekennzeichnet, dass** es sich bei dem pektinhaltigen Material um Zuckerrübenschnitzel, Apfeltrester oder getrocknete Rückstände aus der Orangensaft-, Zitronensaft- und/oder Limonensaft-Herstellung handelt.

6. Verfahren nach einem der Ansprüche 1 bis 5, **dadurch gekennzeichnet, dass** es sich bei dem Enzym A um eine Endopolygalakturonase oder eine Pektinlyase (EC 4.2.2.10) handelt.

7. Verfahren nach einem der Ansprüche 1 bis 6, **dadurch gekennzeichnet, dass** es sich bei dem Enzym B um eine Endopolygalakturonase (EC 3.2.1.15) oder eine Pektinlyase handelt.

8. Pektinhydrolyseprodukte, herstellbar nach einem der Verfahren gemäß einem der Ansprüche 1 bis 7, wobei in den Pektinhydrolyseprodukten der Anteil von Kohlenhydraten mit einem DP-1 an den Gesamtkohlenhydraten des Pektinhydrolysats <25 Gew.-% (bezogen auf die Trockensubstanz) ist.

9. Pharmazeutisches oder diätetisches Präparat, enthaltend ein Pektinhydrolyseprodukt nach Anspruch 8 gegebenenfalls zusammen mit einem pharmazeutisch verträglichen Träger.

10. Verwendung der Pektinhydrolyseprodukte hergestellt nach einem der Verfahren nach einem der Ansprüche 1 bis 7 für die Herstellung eines pharmazeutischen Präparates zur Infektionsbekämpfung.

11. Verwendung nach Anspruch 10, wobei das pharmazeutische Präparat oral verabreicht wird.

12. Verwendung der Pektinhydrolyseprodukte hergestellt nach einem der Verfahren nach einem der Ansprüche 1 bis 7 als Bestandteil von für die menschliche Ernährung bestimmter Nahrungsmitteln.

13. Verwendung der Pektinhydrolyseprodukte hergestellt nach einem der Verfahren nach einem der Ansprüche 1 bis 7 als Tierfutterbestandteil.

14. Verwendung der Pektinhydrolyseprodukte hergestellt nach einem der Verfahren nach einem der Ansprüche 1 bis 7 zur Herstellung eines pharmazeutischen Präparates zur Hemmung von Zell-Zell-Wechselwirkungen und/oder Wechselwirkungen zwischen Zellen und der extrazellulären Matrix bei einem Menschen oder einem Säugetier.

15. Verwendung nach Anspruch 14, wobei die Zell-Zell- und/oder Zell-Matrix-Wechselwirkungen durch auf der Zelloberfläche befindliche Kohlenhydrat-bindende Galectin 3-Moleküle vermittelt werden.

16. Verwendung nach Anspruch 14 oder 15, wobei die Zellen Tumorzellen sind und die Zell-Zell- und/oder Zell-Matrix-Wechselwirkungen für die Entwicklung von humanen oder Säugetier-Tumorerkrankungen verantwortlich sind.

17. Verwendung nach einem der Ansprüche 14 bis 16, wobei die Tumorerkrankungen Prostata-Karzinome, Nieren-Karzinome, Kaposi-Sarkome, Verlaufsformen von chronischer Leukämie, Mammakarzinome, Mamma-Adenokarzinome, Sarkome, Ovarialkarzinome, Rektumkarzinome, Rachenkarzinome, Melanome, Dünndarmtumore, Dickdarmkarzinome, Blasentumore, Mastozytome, Lungenkarzinome, Bronchialkarzinome, Rachen-Plattenepithelkarzinome, Gastrointestinalkarzinome und/oder Magentumore sind.

18. Verwendung nach einem der Ansprüche 14 bis 17, wobei die Pektinhydrolysate die Adhäsion von Tumorzellen hemmen und/oder das Invasionspotential metastasierender Tumorzellen reduzieren.

19. Verwendung nach einem der Ansprüche 14 bis 18, wobei das pharmazeutische Präparat oral verabreicht wird.

20. Verwendung der Pektinhydrolyseprodukte hergestellt nach einem der Verfahren nach einem der Ansprüche 1 bis 7 zur Herstellung eines pharmazeutischen Präparates zur Behandlung von Tumorerkrankungen eines Menschen oder eines Säugetiers.

21. Verwendung nach Anspruch 20, wobei die Tumorerkrankungen auf Zell-Zell-Wechselwirkungen und/oder Wechselwirkungen zwischen Zellen und der extrazellulären Matrix basieren.

22. Verwendung nach Anspruch 20 oder 21, wobei die Zell-Zell- und/oder Zell-Matrix-Wechselwirkungen durch auf der Zelloberfläche befindliche Kohlenhydrat-bindende Galectin 3-Moleküle vermittelt werden.

23. Verwendung nach einem der Ansprüche 20 bis 22, wobei die Tumorerkrankungen Prostata-Karzinome, Nieren-Karzinome, Kaposi-Sarkome, Verlaufsformen von chronischer Leukämie, Mammakarzinome, Mamma-Adenokarzinome, Sarkome, Ovarialkarzinome, Rektumkarzinome, Rachenkarzinome, Melanome, Dünndarmtumore, Dickdarmkarzinome, Blasentumore, Mastozytome, Lungenkarzinome, Bronchialkarzinome, Rachen-Plattenepithelkarzinome, Gastrointestinalkarzinome und/oder Magentumore sind.

24. Verwendung nach einem der Ansprüche 20 bis 23, wobei das pharmazeutische Präparat zur Reduzierung des Tumorwachstums, insbesondere zur Hemmung der Adhäsion von Tumorzellen eingesetzt wird.

25. Verwendung nach einem der Ansprüche 20 bis 24, wobei das pharmazeutische Präparat zur Reduzierung der Metastasenbildung, insbesondere zur Reduzierung des Invasionspotentials von Tumorzellen eingesetzt wird.

26. Verwendung nach einem der Ansprüche 20 bis 25, wobei das pharmazeutische Präparat oral verabreicht wird.

## Claims

1. A method for the manufacture of pectin hydrolysis products, whereby a pectin or pectin-containing plant material in aqueous solution or suspension is treated with a pectin-hydrolyzing enzyme A in a first step of the method and a pectin-hydrolyzing enzyme B in a second step of the method, wherein pectin hydrolysis products having a fraction of galacturonides which contain at least one 4,5-unsaturated galacturonic acid molecule and are esterified with methanol at a rate of ≥ 20% are obtained.

2. A method for the manufacture of pectin hydrolysis products according to claim 1, wherein the liquid hydrolysis products obtained from the second step of the method are treated with an enzyme C in a third step of the method, wherein the enzyme C is a pectin esterase (EC 3.1.1.11).

3. A method for the manufacture of pectin hydrolysis products according to claim 1 or 2, wherein the liquid hydrolysis products obtained from the second or third step of the method are freed from insoluble components by filtration and/or centrifugation, and transferred to a dry form.

4. Method according to any one of the claims 1 to 3, **characterized in that** the pectin used is citrus pectin, apple pectin or sugar beet pectin.

5. Method according to any one of the claims 1 to 4, **characterized in that** the pectin-containing material is sugar beet chips, apple pomace or dried residual material from orange juice, lemon juice and/or lime juice production.

6. Method according to any one of the claims 1 to 5, **characterized in that** enzyme A is an endopolygalacturonase or a pectin lyase (EC 4.2.2.10).

7. Method according to any one of the claims 1 to 6, **characterized in that** enzyme B is an endopolygalacturonase (EC 3.2.1.15) or a pectin lyase.

8. Pectin hydrolysis products that can be manufactured according to any one of the methods according to any one of the claims 1 to 7, whereby, in the pectin hydrolysis products, the fraction of carbohydrates having a DP-1 relative to the total carbohydrates of the pectin hydrolysate is < 25 wt-% (relative to the dry substance).

9. Pharmaceutical or dietetic preparation containing a pectin hydrolysis product according to claim 8, in combination with a pharmaceutically tolerable carrier, if applicable.

10. Use of the pectin hydrolysis products that are manufactured according to any one of the methods according to any one of the claims 1 to 7 for the manufacture of a pharmaceutical anti-infective preparation.

11. Use according to claim 10, whereby the pharmaceutical preparation is administered by the oral route.

12. Use of the pectin hydrolysis products that are manufactured according to any one of the methods according to any one of the claims 1 to 7 as a component of foods designed for human nutrition.

13. Use of the pectin hydrolysis products that are manufactured according to any one of the methods according to any one of the claims 1 to 7 as an animal feed component.

14. Use of the pectin hydrolysis products that are manufactured according to any one of the methods according to any one of the claims 1 to 7 for the manufacture of a pharmaceutical preparation for inhibiting cell-cell interactions and/or interactions between cells and the extracellular matrix in a human or a mammal.

15. Use according to claim 14, whereby the cell-cell and/or cell-matrix interactions are mediated by carbohydrate-binding galectin-3 molecules that are situated on the cell surface.

16. Use according to claim 14 or 15, whereby the cells are tumor cells and the cell-cell and/or cell-matrix interactions are responsible for the development of human or mammal tumor disease.

17. Use according to any one of the claims 14 to 16, whereby the tumor diseases are prostate carcinomas, renal carcinomas, Kaposi's sarcomas, forms of chronic leukemia, mammary carcinomas, mammary adenocarcinomas, sarcomas, ovarian carcinomas, rectal carcinomas, pharyngeal carcinomas, melanomas, small intestinal tumors, large intestinal carcinomas, bladder tumors, mastocytomas, lung carcinomas, bronchial carcinomas, pharyngeal squamous cell carcinomas, gastrointestinal carcinomas and/or gastric tumors.

18. Use according to any one of the claims 14 to 17, whereby the pectin hydrolysates inhibit the adhesion of tumor cells and/or reduce the invasive potential of metastatic tumor cells.

19. Use according to any one of the claims 14 to 18, whereby the pharmaceutical preparation is administered by the oral route.

20. Use of the pectin hydrolysis products that are manufactured according to any one of the methods according to any one of the claims 1 to 7 for the manufacture of a pharmaceutical preparation for treating tumor diseases of a human or a mammal.

21. Use according to claim 20, whereby the tumor diseases are based on cell-cell interactions and/or interactions between cells and the extracellular matrix.

22. Use according to claim 20 or 21, whereby the cell-cell and/or cell-matrix interactions are mediated by carbohydrate-binding galectin-3 molecules that are situated on the cell surface.

23. Use according to any one of the claims 20 to 22, whereby the tumor diseases are prostate carcinomas, renal carcinomas, Kaposi's sarcomas, forms of chronic leukemia, mammary carcinomas, mammary adenocarcinomas, sarcomas, ovarian carcinomas, rectal carcinomas, pharyngeal carcinomas, melanomas, small intestinal tumors, large intestinal carcinomas, bladder tumors, mastocytomas, lung carcinomas, bronchial carcinomas, pharyngeal squamous cell carcinomas, gastrointestinal carcinomas and/or gastric tumors.

24. Use according to any one of the claims 20 to 23, whereby the pharmaceutical preparation is used for reducing the growth of the tumor, in particular for inhibiting the adhesion of tumor cells.

25. Use according to any one of the claims 20 to 24, whereby the pharmaceutical preparation is used for reducing the formation of metastases, in particular for reducing the invasive potential of tumor cells.

26. Use according to any one of the claims 20 to 25, whereby the pharmaceutical preparation is administered by the oral route.

## Revendications

1. Procédé destiné à produire des hydrolysats de pectine, dans lequel une pectine ou une substance végétale contenant de la pectine est traitée dans une solution aqueuse ou en suspension, au cours d'une première étape de procédé, avec une enzyme A hydrolysant la pectine et, au cours d'une deuxième étape de procédé, avec une enzyme B hydrolysant la pectine, les hydrolysats de pectine obtenus présentant une proportion de galacturonides, contenant au moins une molécule d'acide galacturonique insaturé en 4,5 et transestérifié à ≥ 20 % avec du méthanol.

2. Procédé destiné à produire des hydrolysats de pectine selon la revendication 1, dans lequel les hydrolysats fluides obtenus à partir de la deuxième étape du procédé sont traités au cours d'une troisième étape de procédé à l'aide d'une enzyme C, l'enzyme C étant une pectine estérase (EC 3.1.1.11).

3. Procédé destiné à produire des hydrolysats de pectine selon la revendication 1 ou 2, dans lequel les hydrolysats fluides obtenus à partir de la deuxième ou de la troisième étape du procédé sont libérés, par filtration et/ou centrifugation, des composants non-solubles et transformés sous forme sèche.

4. Procédé selon l'une quelconque des revendications 1 à 3, **caractérisé en ce qu'**il s'agit, pour la pectine utilisée, de pectine de citrus, de pectine de pomme ou de pectine de betterave à sucre.

5. Procédé selon l'une quelconque des revendications 1 à 4, **caractérisé en ce qu'**il s'agit, concernant la substance contenant de la pectine, de pulpe de betterave, de marc de pomme ou de résidus secs provenant de la production de jus d'orange, de jus de citron et/ou de jus de citron vert.

6. Procédé selon l'une quelconque des revendications 1 à 5, **caractérisé en ce qu'**il s'agit, dans le cas de l'enzyme A, d'une endopolygalacturonase ou d'une pectine lyase (EC 4.2.2.10).

7. Procédé selon l'une quelconque des revendications 1 à 6, **caractérisé en ce qu'**il s'agit, dans le cas de l'enzyme B, d'une endopolygalacturonase (EC 3.2.1.15) ou d'une pectine lyase.

8. Hydrolysats de pectine pouvant être produits d'après l'un des procédés selon l'une quelconque des revendications 1 à 7, dans lequel la proportion de glucides, dans les hydrolysats de pectine, présentant un DP-1, est < 25 % en poids (rapportée à la substance sèche) rapportée à la teneur totale en glucides des hydrolysats de pectine.

9. Préparation pharmaceutique ou diététique contenant un hydrolysat de pectine selon la revendication 8, le cas échéant avec un excipient pharmaceutiquement compatible.

10. Utilisation des hydrolysats de pectine produits d'après l'un des procédés selon l'une quelconque des revendications 1 à 7 pour la préparation d'une composition pharmaceutique destinée à lutter contre les infections.

11. Utilisation selon la revendication 10 dans laquelle la composition pharmaceutique est administrée par voie orale.

12. Utilisation des hydrolysats de pectine produits d'après l'un des procédés selon l'une quelconque des revendications 1 à 7 sous la forme d'un composant destiné aux denrées alimentaires spécifiques à l'alimentation humaine.

13. Utilisation des hydrolysats de pectine produits d'après l'un des procédés selon l'une quelconque des revendications 1 à 7 en tant que composant de l'alimentation animale.

14. Utilisation des hydrolysats de pectine produits d'après l'un des procédés selon l'une quelconque des revendications 1 à 7 pour la préparation d'une composition pharmaceutique destinée à inhiber les interactions cellule-cellule et/ou les interactions entre des cellules et la matrice extracellulaire chez un homme ou chez un mammifère.

15. Utilisation selon la revendication 14, dans laquelle les interactions cellule-cellule et/ou cellule-matrice sont transmises par des molécules de galectine-3 liant des glucides se trouvant à la surface des cellules.

16. Utilisation selon la revendication 14 ou 15, dans laquelle les cellules sont des cellules cancéreuses et les interactions cellule-cellule et/ou cellule-matrice sont responsables du développement de pathologies cancéreuses chez les hommes ou les mammifères.

17. Utilisation selon l'une quelconque des revendications 14 à 16, dans laquelle les pathologies cancéreuses sont des cancers de la prostate, des cancers du rein, des sarcomes de Kaposi, des formes de développement de leucémie chronique, des cancers du sein, des adénocarcinomes mammaires, des sarcomes, des cancers des ovaires, des cancers du rectum, des cancers du pharynx, des mélanomes, des cancers de l'intestin grêle, des cancers du gros intestin, des tumeurs de la vessie, des mastocytomes, des cancer des poumons, des cancers bronchiques, des carcinomes à cellules squameuses du pharynx, des cancers du tractus gastro-intestinal et/ou des tumeurs de l'estomac.

18. Utilisation selon l'une quelconque des revendications 14 à 17, dans laquelle les hydrolysats de pectine inhibent l'adhérence des cellules cancéreuses et/ou réduisent le potentiel d'invasion des cellules cancéreuses se métastasant.

19. Utilisation selon l'une quelconque des revendications 14 à 18, dans laquelle la préparation pharmaceutique est administrée par voie orale.

20. Utilisation des hydrolysats de pectine produits d'après l'un des procédés selon l'une quelconque des revendications 1 à 7 pour la préparation d'une composition pharmaceutique destinée au traitement des cancers chez un homme ou un mammifère.

21. Utilisation selon la revendication 20, dans laquelle les pathologies cancéreuses reposent sur les interactions cellule-cellule et/ou sur les interactions entre des cellules et la matrice extracellulaire.

22. Utilisation selon la revendication 20 ou 21, dans laquelle les interactions cellule-cellule et/ou cellule-matrice sont transmises par des molécules de galectine-3 liant des glucides se trouvant à la surface des cellules.

23. Utilisation selon l'une quelconque des revendications 20 à 22, dans laquelle les pathologies cancéreuses sont des cancers de la prostate, des cancers du rein, des sarcomes de Kaposi, des formes de développement de leucémie chronique, des cancers du sein, des adénocarcinomes mammaires, des sarcomes, des cancers des ovaires, des cancers du rectum, des cancers du pharynx, des mélanomes, des cancers de l'intestin grêle, des cancers du gros intestin, des tumeurs de la vessie, des mastocytomes, des cancer des poumons, des cancers bronchiques, des carcinomes à cellules squameuses du pharynx, des cancers du tractus gastro-intestinal et/ou des tumeurs de l'estomac.

24. Utilisation selon l'une quelconque des revendications 20 à 23, dans laquelle la préparation pharmaceutique est utilisée pour réduire la croissance tumorale, notamment pour inhiber l'adhérence des cellules cancéreuses.

25. Utilisation selon l'une quelconque des revendications 20 à 24, dans laquelle la préparation pharmaceutique est utilisée pour réduire la formation de métastases, notamment pour réduire le potentiel d'invasion des cellules cancéreuses.

26. Utilisation selon l'une quelconque des revendications 20 à 25, dans laquelle la préparation pharmaceutique est administrée par voie orale.
